(19) Europäisches Patentamt
European Patent Office
Office européen des brevets

(11) Publication number: **0 374 674**
**A2**

# (12) EUROPEAN PATENT APPLICATION

(21) Application number: **89122826.4**

(22) Date of filing: **11.12.89**

(51) Int. Cl.$^5$: **C07D 211/20, C07D 405/12, //A61K31/445**

(30) Priority: **22.12.88 DK 7159/88**

(43) Date of publication of application: **27.06.90 Bulletin 90/26**

(84) Designated Contracting States: **AT BE CH DE ES FR GB GR IT LI LU NL SE**

(71) Applicant: **A/S FERROSAN**
**Sydmarken 5**
**DK-2860 Soeborg(DK)**

(72) Inventor: **Lundbeck, Jane Marie**
**Evas Allé 19**
**DK-2600 Glostrup(DK)**
Inventor: **Everland, Peer**
**Tordisvej 62**
**DK-2880 Bagsvaerd(DK)**
Inventor: **Treppendahl, Svend**
**Frederiksdalsvej 221**
**DK-2830 Virum(DK)**
Inventor: **Jakobsen, Palle**
**langkaer Vaenge 14**
**DK-3500 Vaerlose(DK)**

(74) Representative: **Patentanwälte Grünecker, Kinkeldey, Stockmair & Partner**
**Maximilianstrasse 58**
**D-8000 München 22(DE)**

## (54) Etherification and dealkylation of piperidine derivatives and intermediates.

(57) The invention relates to a novel chemical process for preparing 4-phenylpiperidines having calcium overload blocking activity and antidepressant activity, and to novel intermediates used in that process.

EP 0 374 674 A2

## Etherification and Dealkylation of Piperidine Derivatives and Intermediates

This invention relates to a novel chemical process for preparing 4-phenylpiperidines having calcium overload blocking activity and antidepressant activity, and to novel intermediates used in that process.

Danish patent application no. 5608/87 discloses compounds having the formula (A)

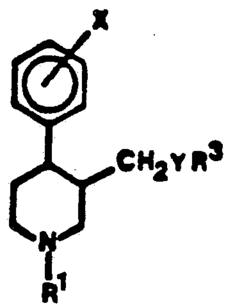

wherein

$R^3$ is 3,4-methylenedioxyphenyl, aryl or heteroaryl which are optionally substituted with one or more $C_{1-6}$-alkyl, $C_{1-6}$-alkoxy, $C_{3-8}$-cycloalkyl, $C_{3-5}$-alkylene or aralkoxy,

$R^1$ is straight or branched $C_{4-8}$-alkyl, $C_{1-8}$-alkoxy-$C_{4-8}$-alkyl, $C_{3-7}$-cycloalkyl, aryloxy-$C_{3-8}$-alkyl, $C_{4-8}$-alkenyl, or $C_{3-8}$-cycloalkylalkyl, or $R^1$ may also be hydrogen or $C_{1-3}$-alkyl, when $R^3$ is aryl, which is substituted with two or more of $C_{1-6}$-alkyl, $C_{1-6}$-alkoxy, $C_{3-8}$-cycloalkyl, aralkoxy, or with $C_{3-5}$-alkylene.

X is hydrogen or halogen, and wherein

Y is O or S

and a salt thereof with a pharmaceutically acceptable acid.

These compounds are disclosed as having calcium overload blocking activity, which makes them useful for the treatment of anoxia, ischemia, migraine and epilepsy.

Danish patent no. 149843 discloses compounds having the formula (B)

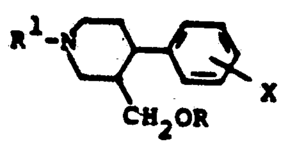

where R is a $C_{1-4}$-alkyl- or $C_{2-4}$-alkynyl group, a tetrahydronaphthyl group or a phenyl group which may be sutstituted 1 or 2 times with halogen or $C_{1-4}$-alkyl-, $C_{1-4}$-alkoxy-, $C_{1-4}$-alkylthio-, nitro-, $C_{1-4}$-acylamino- or methylsulphonyl groups or with a methylenedioxy group, $R^1$ is hydrogen, a 2,2,2-trifluoroethyl group or a $C_{1-4}$-alkyl- or $C_{2-4}$-alkynyl group and X is hydrogen, halogen or a hydroxy-, $C_{1-4}$-alkyl-, $C_{1-4}$-alkoxy-, $C_{1-4}$-trifluoroalkyl-, methylthio- or benzyloxy group, or optically active forms thereof or salts of the compounds with pharmaceutically acceptable acids.

These compounds are disclosed as having 5HT uptake inhibiting activity, which makes the compounds useful as antidepressants.

The compounds of formula (A) are in the Danish patent application no. 5608/87 prepared by alkylating a compound having the formula (C)

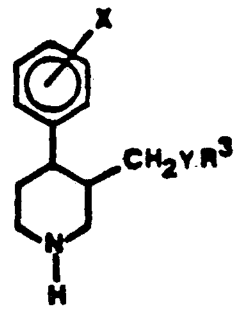

The compounds of Danish patent no. 149843 are made one way or another from an intermediate, having the formula (D)

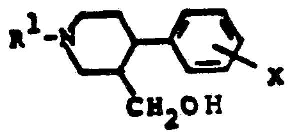

The methods described in above patent application and in above Danish patent all involve intermediates, which preferably should be avoided. One of these intermediates is arecoline, which is a powerful irritant and another intermediate is the compound having the formula (E) 1-methyl-4-phenyl-1,2,3,6-tetrahydropyridine (MPTP). This compound is known to be a very powerful neurotoxine and is a strong promotor of irreversible Parkinsonism (Psychiatry 1, (1979) 249-54, Science, 219 (1983) 979-80).

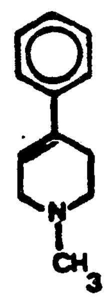

Therefore there is a need to find another process, avoiding this specific compound, for producing the compounds of above Danish patent application no. 5608/87 and Danish patent no. 149843.

The novel process provided with the present invention can be illustrated by the following scheme:

## Scheme I

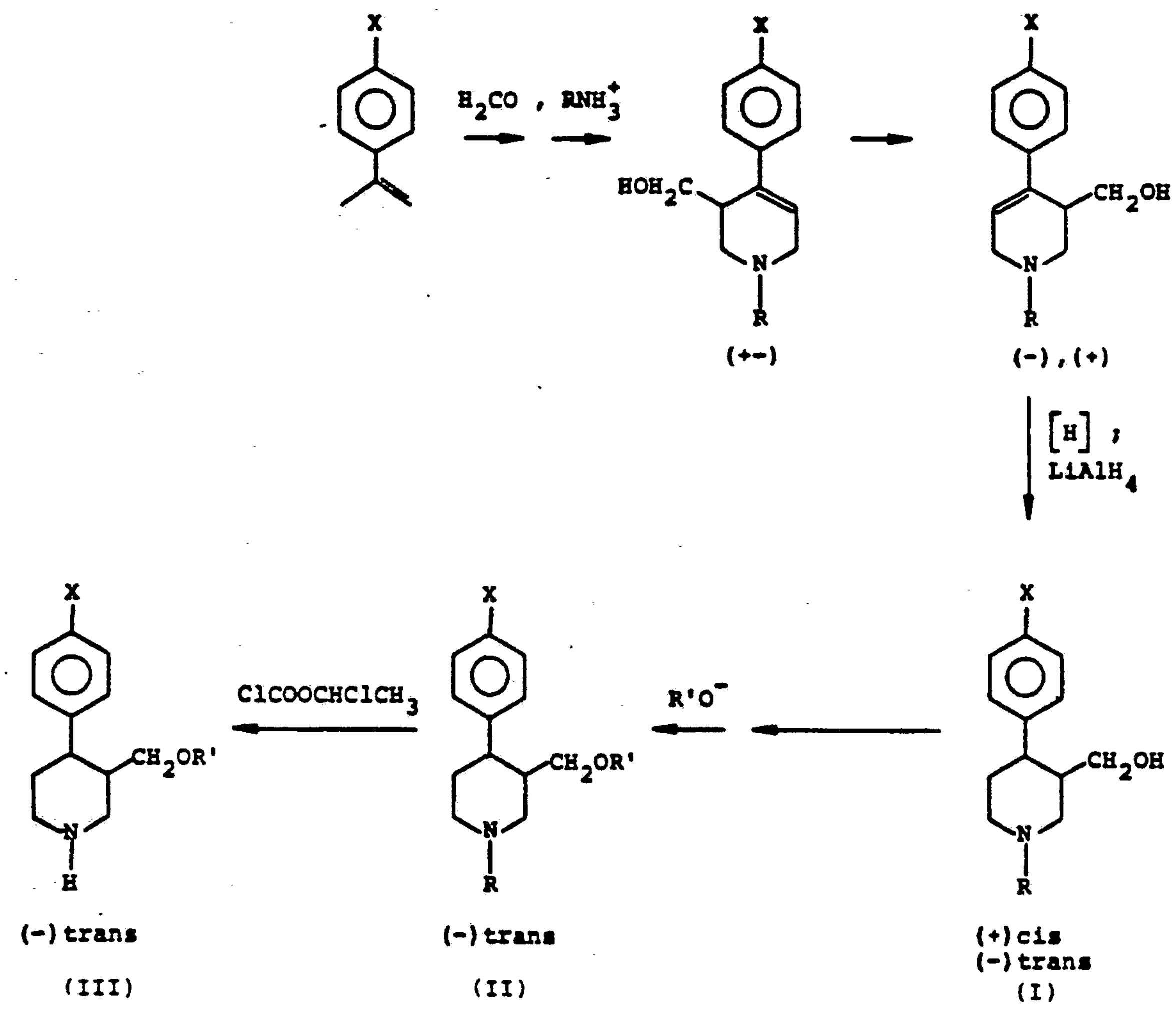


(+-)

(-) , (+)

(-) trans (III)

(-) trans (II)

(+) cis
(-) trans
(I)

The compounds of formula (I) are useful in the preparation of compounds of formula (II) and (III). The compounds of formula (II) are examples of those described in Danish patent application no. 5608/87, and compounds having the formula (III) are examples of the compounds disclosed in Danish patent 149843.

The following examples illustrate the novel process of the present invention, the novel intermediates of the present invention and the utility of the novel intermediates of the present invention.

### EXAMPLE 1

(+-)-1-butyl-3-hydroxymethyl-4-phenyl-1,2,3,6-tetrahydropyridine hydrochloride

Butylamine (317 g) was mixed with concentrated hydrochloric acid (350 ml) keeping the temperature below 40°C. The pH of the solution was adjusted between 4-5. Formaldehyde (624 ml, 39%) was then added, and the solution heated to 50-60°C for 1 h. Subsequently 1-methylstyrene (563 ml) was added in five portions keeping the temperature in the reaction around 80°C. After the styrene had been added the reaction mixture was refluxed for five h. It was cooled to room temperature and extracted twice with toluene (500 ml and 200 ml) and made alkaline (pH = 8-9) with sodium hydroxide (9M) (430 ml). The tetrahydropyridine base was extracted twice with toluene (500 ml and 250 ml). Concentrated hydrochloric acid (826 ml) and formaline (454 ml, 39%) was added to the toluene extract and the tetrahydropyridine base was extracted from the toluene phase. The toluene was discharged. The aqueous solution was heated to 80°C for 8 h, left overnight, made alkaline (pH = 8-9) with sodium hydroxide (1000 ml, 9 M) and extracted twice with toluene (1500 ml and 250 ml). The toluene ex tract was extracted once with 0.5 M hydrochloric acid (625 ml) and 6 times with 0.5 M hydrochloric acid (600 ml). The first extract was discharged and the following six extracts were pooled, made alkaline (pH = 8-9) with sodium hydroxide (9M) and extracted twice with toluene (500 ml and 250 ml). The toluene extract was dried over potassium carbonate and evaporated. The residue was redissolved in acetone (1000 ml) and precipitated with concentrated hydrochloric acid (160 ml, pH = 2-3). Yield 227 g, m.p. 180-181°C. (1).

(+-)-1-butyl-4-(4-fluorophenyl)-3-hydroxymethyl-1,2,3,6-tetrahydropyridine hydrochloride was prepared in an analogous way. The product was identified by $^1$H-NMR and MS. M.p. 172-175°C. (2).

(+-)-3-hydroxymethyl-1-pentyl-4-phenyl-1,2,3,6-tetrahydropyridine hydrochloride was prepared in an analogous way. The product was identified by $^1$H-NMR and MS. M.p. 189-190.5°C. (3).

(+-)-1-benzyl-3-hydroxymethyl-4-phenyl-1,2,3,6-tetrahydropyridine hydrochloride was prepared in an analogous way. The product was identified by $^1$H-NMR and MS. M.p. 182.5-192°C. (4).

(+-)-4-(4-fluorophenyl)-3-hydroxymethyl-1-pentyl-1,2,3,6-tetrahydropyridine hydrochloride was prepared in an analogous way. The identify was confirmed by $^1$H-NMR and MS. M.p. 223-227°C. (5).

## EXAMPLE 2

(+-)trans-1-butyl-3-hydroxymethyl-4-phenyl-piperidine

1 g (0.26 mol) $LiAlH_4$ was mixed with dry THF. 2.6 g (0.011 mol) (1) (free amine) dissolved 100 ml dry THF was added under $N_2$. After the addition the reaction mixture was refluxed for 5 h. Water was added very carefully. The water layer was separated and washed with 3x200 ml ether. The organic layers were mixed, dried over $MgSO_4$ and evaporated in vacuo. Resulting in 2.5 g oil. The oil was dissolved in $CH_2Cl_2$ and 1.3 g title compound precipitated as colourless crystals by addition of hexane. M.p. 112.5-113°C. (6).

(+-)trans-4-(4-fluorophenyl)-3-hydroxymethyl-1-pentylpiperidine was prepared in the same way. M.p. 131-2°C. (7).

(-)cis-1-butyl-3-hydroxymethyl-4-phenyl-piperidine

(+)-1-butyl-3-hydroxymethyl-4-phenyl-1,2,3,6-tetrahydropyridine hydrochloride (486 g) was dissolved in ethanol (500 ml) acetic acid (50 ml) and water (500 ml). Palladium on charcoal, 5% Engelhardt with 50% water (20 g) was added, and the hydrogenation was carried out at about 50°C and 70 atm. for 8 h. The catalyst was removed by filtration. Water (500 ml), toluene (1 l) and sodium hydroxide (350 ml, 9 M) was added. The toluene was separated and the water phase extracted twice with toluene (2 x 200 ml). The toluene extract was dried over potassium carbonate, filtered and evaporated under reduced pressure leaving an oily residue, which was crystallized from p-ether. Yield 265 g, m.p. 55,3-56°C, $[\alpha]_D^{20}$ = -73.0° (c = 5% in methanol). The identity was confirmed by $^1$H-NMR and MS. (8).

(+)cis-1-butyl-4-(4-fluorophenyl)-3-hydroxymethyl-piperidine was prepared in an analogous way. M.p. 58-59°C, $[\alpha]_D^{20}$ = 65.8° (c = 5% in methanol). The identity was confirmed by $^1$H-NMR and MS. (9).

(+)cis-3-hydroxymethyl-1-pentyl-4-phenyl-piperidine was prepared in an analogous way. M.p. 122-122.5°C, $[\alpha]_D^{20}$ = +57.5° (c = 2% in methanol). The identity was confirmed by $^1$H-NMR and MS. (10).

(+)cis-4-(4-fluorophenyl)-3-hydroxymethyl-1-pentyl-piperidine was prepared in an analogous way. M.p. 55.3-56°C, $[\alpha]_D^{20}$ = +63.6° (c = 5% in methanol). The identity was confirmed by $^1$H-NMR and MS. (11).

## EXAMPLE 3

(+-)trans-3-benzenesulfonylmethyl-1-butyl-4-phenylpiperidine

5 g 0.020 mol) (6) was dissolved in toluene and MIBC. 3.88 g (0.022 mol) benzenesulfonyl chloride and 4.5 ml 50% NaOH was added. Reaction time 4 h at room temperature. 4N NaOH was added, and the organic phase was separated. The water phase was washed with ether. The organic layer was washed with 4N NaOH, then dried and evaporated, resulting in 9 g (still containing some toluene) as an oil. The identity was confirmed by $^1$H-NMR. (12).

(+-)trans-3-benzenesulfonylmethyl-4-(4-fluorophenyl)-1-pentyl-piperidine was prepared in the same way. The product was an oil, which was identified by $^1$H-NMR. (13).

## EXAMPLE 4

(+-)trans-4-phenyl-3-(3,4-methylenedioxyphenoxymethyl)-1-butyl-piperidine

7 g (0.018 mol) (12) was dissolved in toluene and MIBC. 3.0 g (0.022 mol) sesamol and 4 ml 50% NaOH was added.

The reaction mixture was refluxed for 5 h. The organic layer was washed with 4N NaOH, dried over $MgSO_4$ and evaporated, yielding an oil. The oil was chromatographed on a silicagel column with $CH_2Cl_2$/methanol (9:1) as eluent, giving 6.1 g as an oil. The oil was dissolved in acetone and 1 eq. of oxalic acid was added. Recrystallization of the resulting precipitate gave 7.1 g as the oxalate salt. M.p. 157-8°C. This oxalate gave 4.66 g free amine as an oil, by extraction with 4N NaOH/ether. (14).

(+-)trans-4-(4-fluorophenyl)-3-(3,4-methylenedioxyphenoxymethyl)-1-pentyl-piperidine was prepared in the same way. The hydrochloride salt was isolated. M.p. 139-139.8°C. (15).

## EXAMPLE 5

(+-)trans-4-phenyl-3-(3,4-methylenedioxyphenoxymethyl)-piperidine

1.5 g (0.0039 mol) (14) was dissolved in dry 1,2-dichloromethane. 0.84 g (0.0059 mol) 1-chloroethylchloroformate was added slowly under $N_2$ and with rapid stirring at 0°C. The temperature was kept at 0°C for 15 min. The reaction mixture was left at 0°C for 15 min., then the reaction mixture was allowed to warm to room temperature, and subsequently refluxed for 30 min. Then the volume was reduced to one third, by evaporation. Methanol was added, and the mixture refluxed for 1.5 h. The reaction mixture was evaporated *in vacuo*, giving an oil. The oil was chromatographed on a silica column with $CH_2Cl_2$/methanol (9:1) as eluent giving 0.98 g of the hydrochloride of the title compound. M.p. 176-177°C. (16).

(+-)trans-4-(4-fluorophenyl)-3-(3,4-methylenedioxyphenoxymethyl)-piperidine was prepared in the same way. The hydro chloride salt was isolated. M.p. 98°C. (17).

## EXAMPLE 6

(+)-1-butyl-3-hydroxymethyl-4-phenyl-1,2,3,6-tetrahydropyridine-(-)-dibenzoyl tartrate

(+-)-1-butyl-3-hydroxymethyl-4-phenyl-1,2,3,6-tetrahydropyridine hydrochloride (20 g) was dissolved in water (80 ml). The solution was made alkaline with sodium hydroxide (9 M), and extracted with toluene (80 ml). The extract was dried over potassium carbonate, filtrated and evaporated under reduced pressure,

leaving the tetrahydropyridine base. The base was dissolved in ethanol (50 ml) and added to a hot solution of (-)-dibenzoyltartaric acid (13.6 g) in ethanol (50 ml). Finally formic acid (1.7 ml) was added. The mixture was cooled and stirred, to crystallize the salt. The precipitate was filtered off, washed with ethanol and dried. Yield 12 g, m.p. 150-152.5°C and $[\alpha]_D^{20}$ = -39.2° (c = 2% in methanol). (18).

(-)-1-butyl-4-(4-fluorophenyl)-3-hydroxymethyl-1,2,3,6-tetrahydropyridine-(-)-dibenzoyl tartrate was prepared in an analogous way. M.p. 146-148°C and $[\alpha]_D^{20}$ = -101.7° (c = 2% in methanol). (19).

(-)-3-hydroxymethyl-1-pentyl-4-phenyl-1,2,3,6-tetrahydropyridine-(-)-dibenzoyl tartrate was prepared analogously. M.p. 101.5-103°C and $[\alpha]_D^{20}$ = -94.1°C (c = 2% in methanol). (20).

(-)-4-(4-fluorophenyl)-3-hydroxymethyl-1-pentyl-1,2,3,6-tetrahydropyridine-(-)-dibenzoyl tartrate was prepared analogously. M.p. 128.5-130°C and $[\alpha]_D^{20}$ = -95.4° (c = 2% in methanol). (21).

## EXAMPLE 7

(+)-1-butyl-3-hydroxymethyl-4-phenyl-1,2,3,6-tetrahydropyridine

(+)-1-butyl-3-hydroxymethyl-4-phenyl-1,2,3,6-tetrahydropyridine-(-)-dibenzoyl tartrate (30 g) was suspended in water (50 ml). The slurry was made alkaline with saturated sodium carbonate solution and extracted with toluene (100 ml). The extract was dried over potassium carbonate, filtered and evaporated under reduced pressure. The residue was crystallized from p-ether. Yield 8 g, m.p. 70-71°C and $[\alpha]_D^{20}$ = +109.6° (c = 5% in methanol). (22)

(-)-1-butyl-4-(4-fluorophenyl)-3-hydroxymethyl-1,2,3,6-tetrahydropyridine was prepared in the same way. M.p. 80-81°C, $[\alpha]_D^{20}$ = -112.4° (c = 2% in methanol). (23).

(-)-3-hydroxymethyl-1-pentyl-4-phenyl-1,2,3,6-tetrahydropyridine was prepared analogously. M.p. 41-41.2°C and $[\alpha]_D^{20}$ = -108.3° (c = 5% in methanol). (24).

(-)-4-(4-fluorophenyl)-3-hydroxymethyl-1-pentyl-1,2,3,6-tetrahydropyridine was prepared in an analogous way. M.p. 38.3-38.9°C and $[\alpha]_D^{20}$ = -99.0° (c = 5% in methanol). (25).

## Claims

1. A compound having the formula

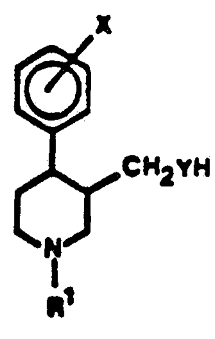

wherein $R^1$ is $C_{2-6}$-alkyl, $C_{1-8}$-alkoxy-$_{4-8}$-alkyl, $C_{3-7}$-cycloalkyl, aryloxy-$C_{3-8}$-alkyl, $C_{4-8}$-alkenyl, or $C_{3-8}$-cycloalkylalkyl;
X is hydrogen or halogen, and
Y is O or S.

2. A process for the preparation of a compound having the formula

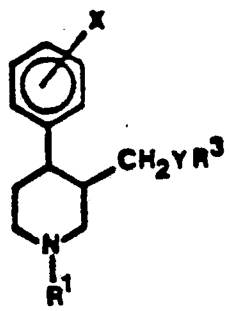

wherein $R^1$, X and Y have the meanings set forth in claim 1 and wherein $R^3$ is 3,4-methylenedioxyphenyl, aryl or heteroaryl which are optionally substituted with one or more $C_{1-6}$-alkyl, $C_{1-6}$-alkoxy, $C_{3-8}$-cycloalkyl, $C_{3-5}$-alkylene or aralkoxy, CHARACTERIZED in reacting a reactive derivative of a compound of claim 1 with a reactive derivative of a compound having the formula $R^3OH$ wherein $R^3$ has the meaning set forth above.

3. A process for the preparation of a compound having the formula

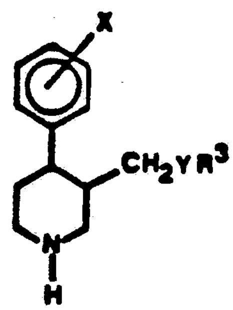

wherein $R^3$, Y and X have the meanings set forth in claim 1 and 2, CHARACTERIZED in dealkylating a compound having the formula

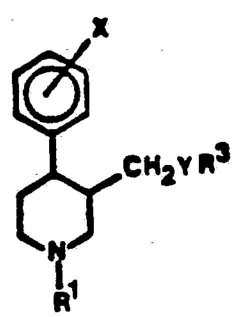

wherein $R^1$, $R^3$, Y, X have the meanings set forth in claim 1 and 2.